# EUROPEAN PATENT APPLICATION

(11) **EP 4 082 596 A1**
(43) Date of publication of application: **02.11.2022**
(21) Application number: 20906971.5
(22) Date of filing: 17.12.2020
(51) Int. Cl.: A61M 37/00

(54) **MICRONEEDLE ARRAY PRODUCTION METHOD**

(30) Priority: 26.12.2019 JP 2019236672
(71) Applicant: FUJIFILM Corporation, Tokyo 106-8620 (JP)
(72) Inventor: USA, Toshihiro, Ashigarakami-gun, Kanagawa 258-8577 (JP); SEKIZAWA, Yasuhiro, Ashigarakami-gun, Kanagawa 258-8577 (JP); TAMAKI, Kenichiro, Ashigarakami-gun, Kanagawa 258-8577 (JP); CHAI, Satoshi, Ashigarakami-gun, Kanagawa 258-8577 (JP); IKEDA, Kozue, Ashigarakami-gun, Kanagawa 258-8577 (JP)
(74) Representative: Klunker IP Patentanwälte PartG mbB
(86) International application number: PCT/JP2020/047178
(87) International publication number: WO 2021/132022

(57) **Abstract**

Provided is a method for manufacturing a microneedle array, which enables efficient and stable manufacturing of a microneedle array including a needle-shaped protrusion having a constricted shape. The method for manufacturing a microneedle array includes: a first cutting step of forming a needle-shaped protrusion by cutting a base material by a first cutting tool; a second cutting step of forming a constricted shape in the needle-shaped protrusion by cutting a part of the needle-shaped protrusion by a second cutting tool different from the first cutting tool; a mold forming step of molding, from a plate precursor manufactured through the first cutting step and the second cutting step, a resin mold having a needle-shaped hole which has an inverted shape of the plate precursor; a first array manufacturing step of filling the needle-shaped hole of the resin mold with a drug solution and then drying the drug solution; a second array manufacturing step of filling the needle-shaped hole with a base material solution and drying the base material solution, after the first array manufacturing step; and a peeling step of peeling, from the resin mold, a microneedle array manufactured by the resin mold.

## Description

### BACKGROUND OF THE INVENTION

### 1. Field of the Invention

The present invention relates to a method for manufacturing a microneedle array.

### 2. Description of the Related Art

In recent years, a microneedle array has been known as a novel dosage form that enables administration of drugs such as insulin, vaccines, and human growth hormone (hGH) into the skin without pain. A self-dissolving microneedle array is an array of microneedles (also referred to as fine needles, or small needles) which contain drugs and are biodegradable. By attaching this microneedle array to the skin, each microneedle pierces the skin, and these microneedles are absorbed in the skin. The drugs contained in each microneedle can be administered into the skin. The microneedle array is also called a transdermal absorption sheet.

The microneedle array is required to provide rapid, simple, and stable administration of the drugs in administration. Therefore, in order to allow a part of the microneedle array to remain in the skin after puncturing the skin, it has been proposed to provide a step in a part of the microneedle array to form a constricted shape or the like (JP2003-238347A and JP2018-079127A).

### SUMMARY OF THE INVENTION

However, it is not easy to manufacture a microneedle array having a stably constricted shape by an X-ray lithography method of JP2003-238347A. In addition, in a method of JP2018-079127A, a process is complicated because a microneedle manufactured in a different process is inserted into a liquid member to manufacture a microneedle array having an arrowhead shape. Efficient manufacturing of the microneedle array is not easy.

The present invention has been made in view of the above-described circumstances, and an object thereof is to provide a method for manufacturing a microneedle array, which enables efficient and stable manufacturing of a microneedle array comprising a needle-shaped protrusion having a constricted shape.

A method for manufacturing a microneedle array of a first aspect comprises: a first cutting step of forming a needle-shaped protrusion by cutting a base material by a first cutting tool; a second cutting step of forming a constricted shape in the needle-shaped protrusion by cutting a part of the needle-shaped protrusion by a second cutting tool different from the first cutting tool; a mold forming step of molding, from a plate precursor manufactured from the base material through the first cutting step and the second cutting step, a resin mold having a needle-shaped hole which has an inverted shape of the plate precursor; a first array manufacturing step of filling the needle-shaped hole of the resin mold with a drug solution and then drying the drug solution; a second array manufacturing step of filling the needle-shaped hole with a base material solution and drying the base material solution, after the first array manufacturing step; and a peeling step of peeling, from the resin mold, a microneedle array manufactured by the resin mold. According to the first aspect, the microneedle array comprising the needle-shaped protrusion can be efficiently and stably manufactured.

In the method for manufacturing a microneedle array of a second aspect, a minimum diameter of the constricted shape is 80 µm or more and 140 µm. According to the second aspect, the preferred minimum diameter of the constricted shape is limited.

In the method for manufacturing a microneedle array of a third aspect, the resin mold is made of silicone rubber. According to the third aspect, the microneedle array having the needle-shaped protrusion having the constricted shape and the plate precursor can be peeled from the resin mold.

In the method for manufacturing a microneedle array of a fourth aspect, the resin mold is molded by a liquid injection molding method. According to the fourth aspect, a preferred method for forming the resin mold is defined.

In the method for manufacturing a microneedle array of a fifth aspect, the constricted shape is formed in a range of 300 µm or more and 500 µm or less from a tip of the needle-shaped protrusion. According to the fifth aspect, the constricted shape can be formed in 300 µm or more and 500 µm or less from the tip of the needle-shaped protrusion of the microneedle array.

In the method for manufacturing a microneedle array of a sixth aspect, the second cutting tool has a cutting edge angle of 30° or more. According to the sixth aspect, since the constricted shape of 30° or more can be formed on the needle-shaped protrusion of the microneedle array, the needle-shaped protrusion is easily broken.

In the method for manufacturing a microneedle array of a seventh aspect, the first cutting tool and the second cutting tool revolve while rotating. According to the seventh aspect, a preferred operation of the first cutting tool and the second cutting tool is defined.

In the method for manufacturing a microneedle array of an eighth aspect, a drug contained in the drug solution is at least one selected from the group consisting of a peptide, a protein, a nucleic acid, a polysaccharide, a vaccine, a pharmaceutical compound, and a cosmetic component. According to the eighth aspect, the preferred type of drug is defined.

According to the method for manufacturing a plate precursor according to an aspect of the present invention, it is possible to efficiently and stably manufacture a microneedle array including a needle-shaped protrusion having a constricted shape.

### BRIEF DESCRIPTION OF THE DRAWINGS

Fig. 1 is a flowchart of a method for manufacturing a microneedle array.
Fig. 2 is a diagram for describing a first cutting step.
Fig. 3 is a diagram for describing the first cutting step.
Fig. 4 is a diagram for describing the first cutting step.
Fig. 5 is a diagram for describing a second cutting step.
Fig. 6 is a diagram for describing the second cutting step.
Fig. 7 is a diagram for describing the second cutting step.
Fig. 8 is a photomicrograph of a needle-shaped protrusion of a plate precursor.
Fig. 9 is a diagram for describing a resin mold manufacturing step.
Fig. 10 is a diagram for describing the resin mold manufacturing step.
Figs. 11A and 11B are diagrams for describing a first array manufacturing step.
Figs. 12A and 12B are diagrams for describing a second array manufacturing step.
Fig. 13 is a view for describing a peeling step.
Fig. 14 is a photomicrograph of the needle-shaped protrusion of the microneedle array.

### DESCRIPTION OF THE PREFERRED EMBODIMENTS

Hereinafter, preferred embodiments of the present invention will be described with reference to the accompanying drawings. The present invention will be described by the following preferred embodiments. Modifications can be made by various methods without departing from the scope of the present invention, and other embodiments than the present embodiment can also be used. Therefore, all modifications within the scope of the present invention are included in the scope of claims.

Here, in the figures, portions indicated by the same symbols are similar elements having similar functions. In addition, in the present specification, in a case where a numerical value range is represented by using "to", the numerical value range includes the numerical values of the upper limit and the lower limit indicated by "to".

### <Method for Manufacturing Microneedle Array>

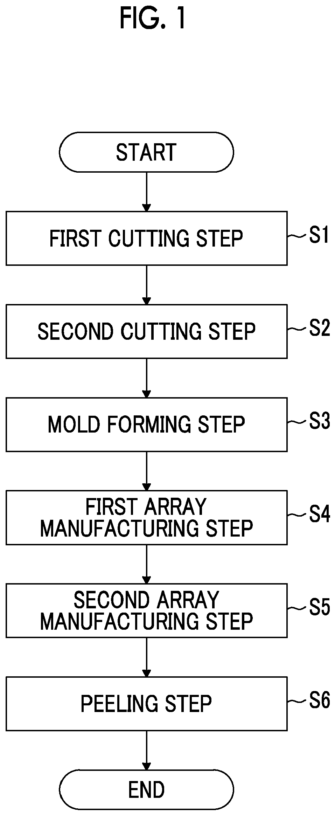

Fig. 1 is a flowchart of a method for manufacturing a microneedle array. As shown in Fig. 1, the method for manufacturing a microneedle array comprises a first cutting step (step S1), a second cutting step (step S2), a mold forming step (step S3), a first array manufacturing step (step S4), a second array manufacturing step (step S5), and a peeling step (step S6).

Next, each step will be described.

### (First Cutting Step)

In the first cutting step, a needle-shaped protrusion is formed by cutting a base material by a first cutting tool (step S1).

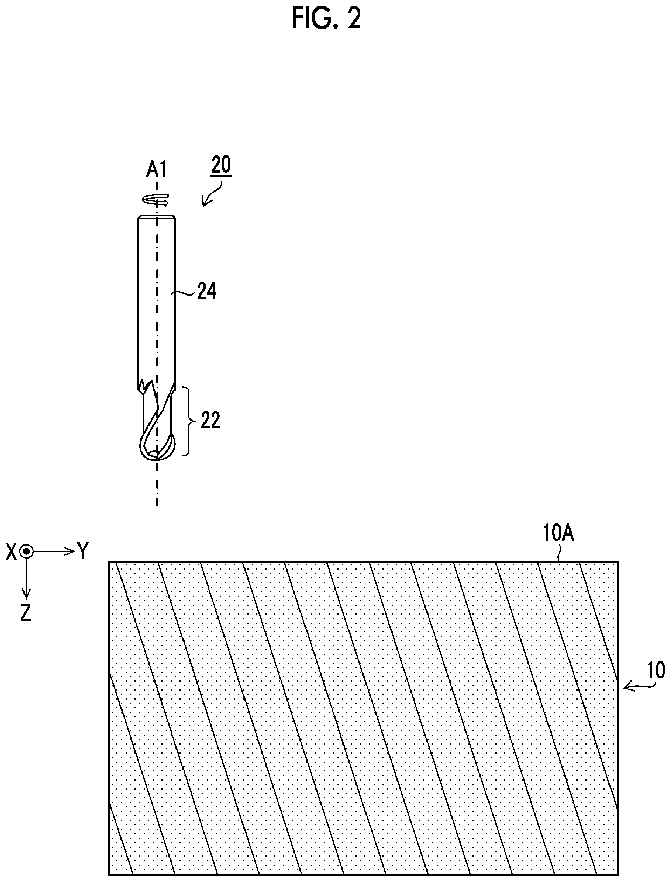

As shown in Fig. 2, a base material 10 and a first cutting tool 20 are prepared in order to manufacture a plate precursor to be described below. The base material 10 has a flat surface 10A to be cut. As a material of the base material 10, metal, cemented carbide, or ceramic can be suitably applied. As the metal, iron, aluminum, stainless steel, Ni plating, Cu plating, brass, titanium, and the like can be suitably applied. Note that the material of the base material 10 is not limited to the above materials. The base material 10 has a substantially rectangular parallelepiped shape.

The first cutting tool 20 comprises a first blade 22 and a shank 24 for holding the first blade 22. The first cutting tool 20 is attached to a spindle (not shown) and can rotate around a tool axis A1.

As a material of the first blade 22, carbide metal, monocrystalline diamond, polycrystalline diamond, cubic boron nitride (CBN), or sintered diamond (polycrystalline diamond (PCD)) can be suitably applied.

As the first cutting tool 20, for example, a ball end mill having a blade at a tip can be applied. The ball end mill has a spherical blade at the tip. The first cutting tool 20 is attached to a drive unit and is movable in an X direction, a Y direction, and a Z direction.

The first cutting tool 20 and the base material 10 are aligned. In the alignment, a position of the first cutting tool 20 is adjusted in consideration of a position of a needle-shaped protrusion 14 (not shown) to be formed on the base material 10.

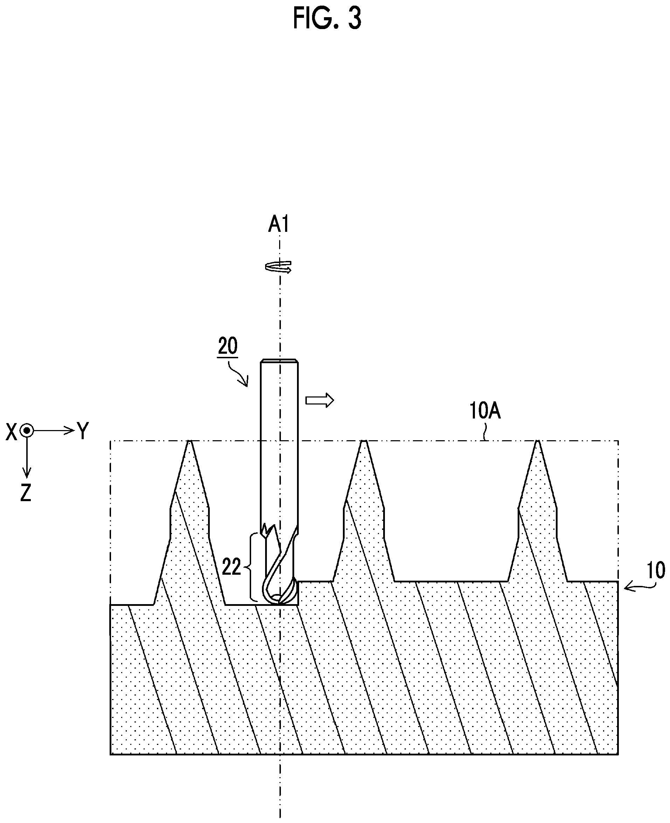

As shown in Fig. 3, the first cutting tool 20 moves in the Z direction. The first cutting tool 20 moves in the XY direction while rotating in a state where the position in the Z direction is fixed, and cuts the base material 10.

In a case where the first cutting tool 20 finishes cutting in the XY direction, the first cutting tool 20 moves slightly in the Z direction. The first cutting tool 20 moves in the XY direction while rotating in a state where the position in the Z direction is fixed, and cuts the base material 10.

The first cutting tool 20 repeats movement in the Z direction, position fixing, and movement in the XY direction, and successively advances machining of the base material 10 in the Z direction.

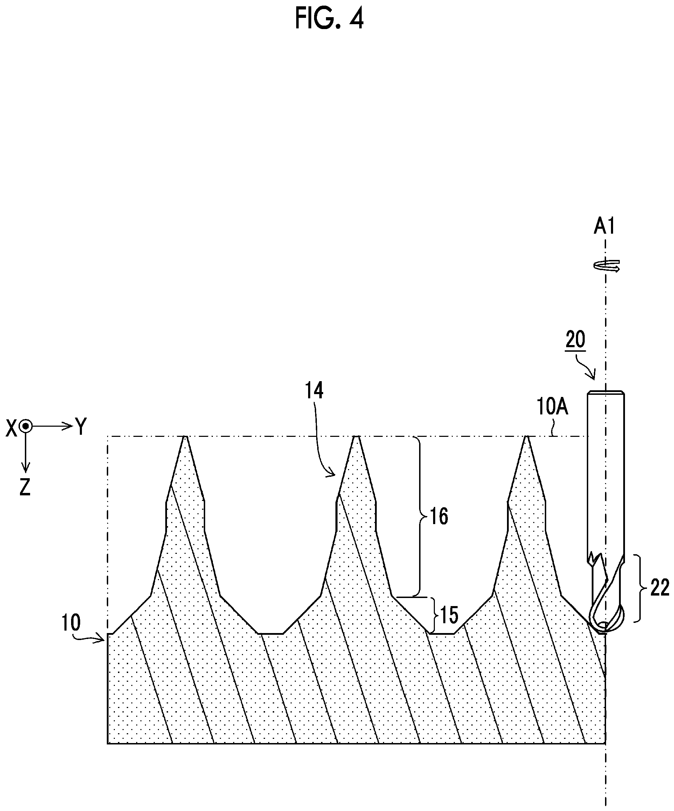

As shown in Fig. 4, the first cutting tool 20 moves in a direction away from the base material 10 after formation of the needle-shaped protrusion 14.

A necessary number of needle-shaped protrusions 14 are formed by the first cutting tool 20. The needle-shaped protrusion 14 has substantially the same shape as an outer shape (excluding a constricted shape 117) of a needle-shaped protrusion 114 formed in a microneedle array 100 to be described below. Although a height of the needle-shaped protrusion 14 is not particularly limited, the height is 800 µm or more and 3000 µm or less, and preferably 1000 µm or more and 2000 µm or less.

### (Second Cutting Step)

In the second cutting step, a constricted shape is formed on the needle-shaped protrusion by cutting a part of the needle-shaped protrusion by a second cutting tool different from the first cutting tool (step S2).

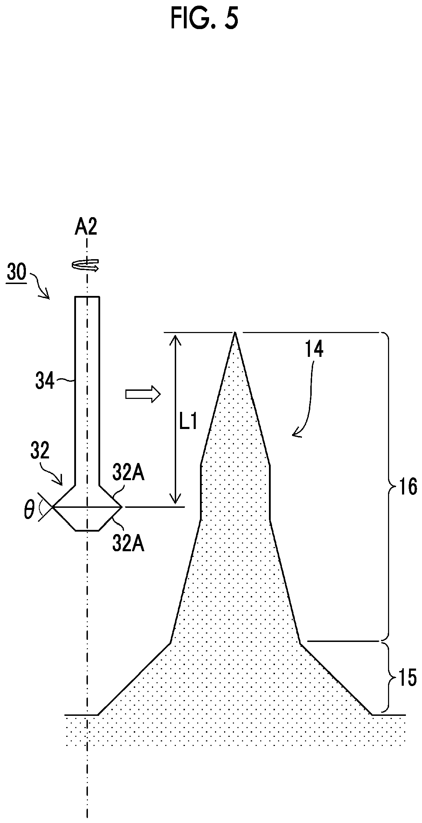

As shown in Fig. 5, a second cutting tool 30 different from the first cutting tool 20 is prepared. The second cutting tool 30 comprises a second blade 32 and a support member 34 for holding the second blade 32. The second cutting tool 30 is attached to a spindle (not shown) and can rotate around a tool axis A2.

As a material of the second blade 32, carbide metal, monocrystalline diamond, polycrystalline diamond, cubic boron nitride (CBN), or sintered diamond (polycrystalline diamond (PCD)) can be suitably applied.

An outer edge 32A of the second blade 32 has a shape following an outer shape of the constricted shape 117 formed in the microneedle array 100 to be described below. A cutting edge angle θ of the second blade 32 is preferably 30° or more, and more preferably 60° or more. In addition, the cutting edge angle θ is preferably 150°C or lower, and more preferably 120°C or lower. In the embodiment, the cutting edge angle θ is 90°. A corner portion of the outer edge 32A of the second blade 32 is set at a distance L1 of 300 µm or more and 500 µm or less from the tip of the needle-shaped protrusion 14. The second cutting tool 30 moves toward the needle-shaped protrusion 14.

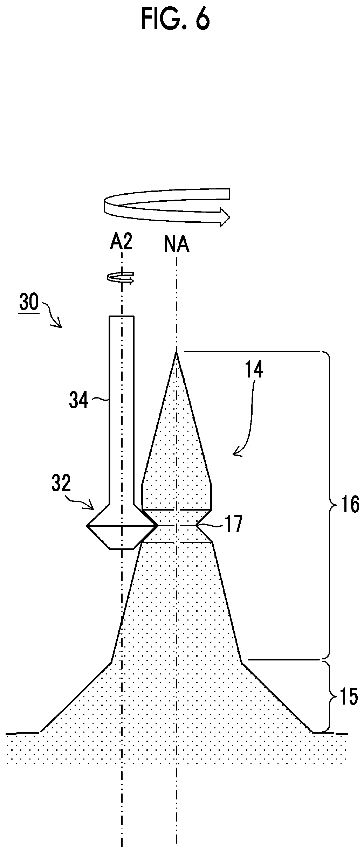

As shown in Fig. 6, the second cutting tool 30 comes into contact with the needle-shaped protrusion 14 while rotating around the tool axis A2, and revolves around an axis NA with a predetermined distance as a revolution radius. The second cutting tool 30 cuts a part of the needle-shaped protrusion 14, thereby forming a constricted shape 17 on the needle-shaped protrusion 14.

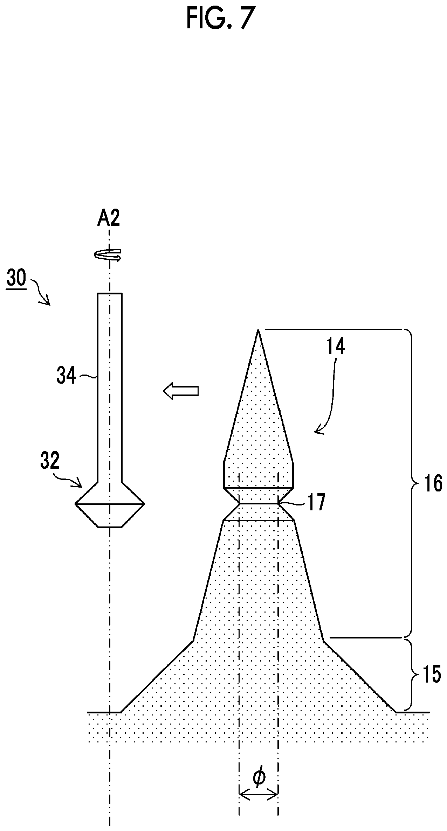

As shown in Fig. 7, the second cutting tool 30 moves in a direction away from the needle-shaped protrusion 14 after formation of the constricted shape 17. The second cutting tool 30 further moves in a direction away from the base material 10. The second cutting tool 30 cuts the needle-shaped protrusions 14 until the constricted shape 17 is formed on all the needle-shaped protrusions 14. The minimum diameter ϕ of the constricted shape 17 is preferably 80 µm or more and 140 µm or less. The constricted shape 17 is formed as the constricted shape 117 of the microneedle array 100 to be described below.

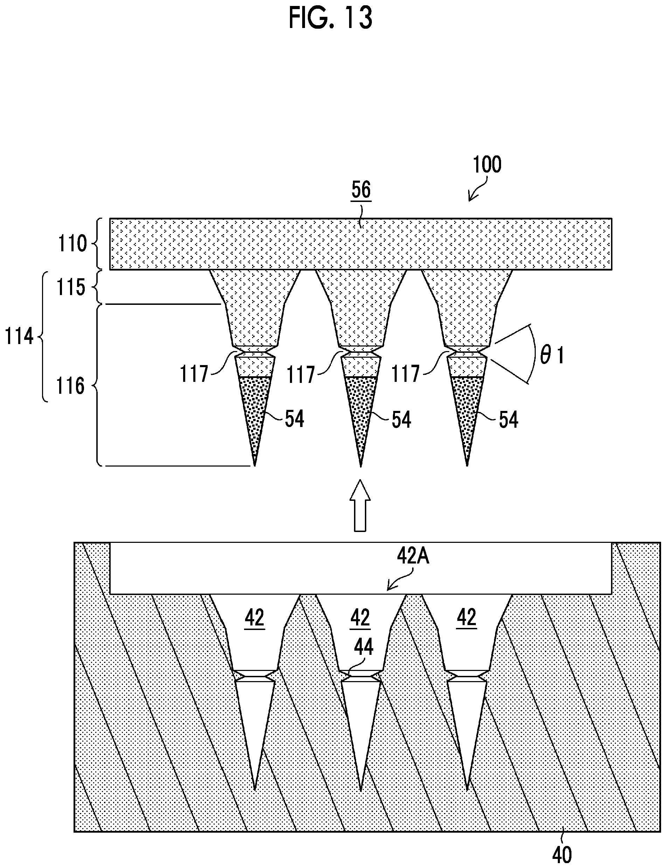

In a case where the constricted shape 17 is formed in a range of 300 µm or more and 500 µm or less, the constricted shape 117 is formed at the same position of the needle-shaped protrusion 114 of the microneedle array 100 (see Fig. 13). The position corresponds to a depth of the microneedle array 100 in a case where the microneedle array 100 punctures the skin. By breaking the needle-shaped protrusion 114 at this position, the drug can be stably administered.

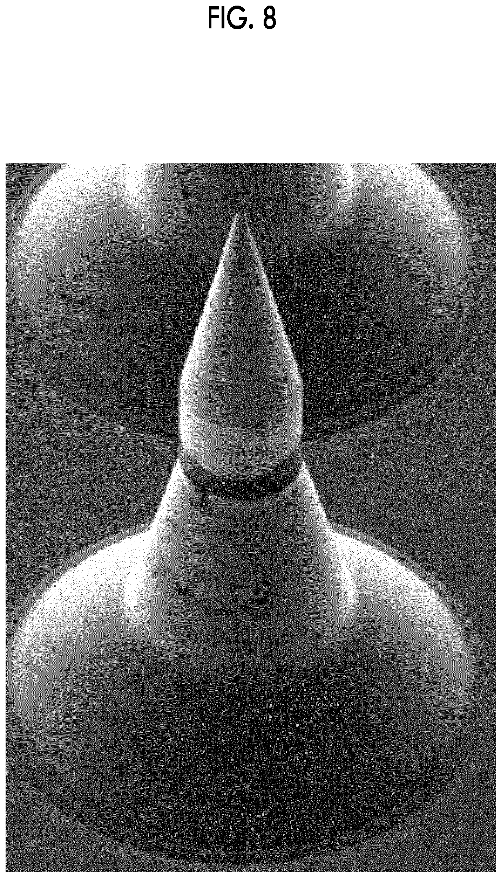

Fig. 8 is a photomicrograph of the needle-shaped protrusion after the second cutting step. According to the photomicrograph, it can be confirmed that the constricted shape is formed on the needle-shaped protrusion by the second cutting tool.

### (Mold Forming Step)

In the mold forming step, from the plate precursor manufactured from the base material through the first cutting step (step S1) and the second cutting step (step S2), a resin mold having a needle-shaped hole which has an inverted shape of the plate precursor is molded (step S3).

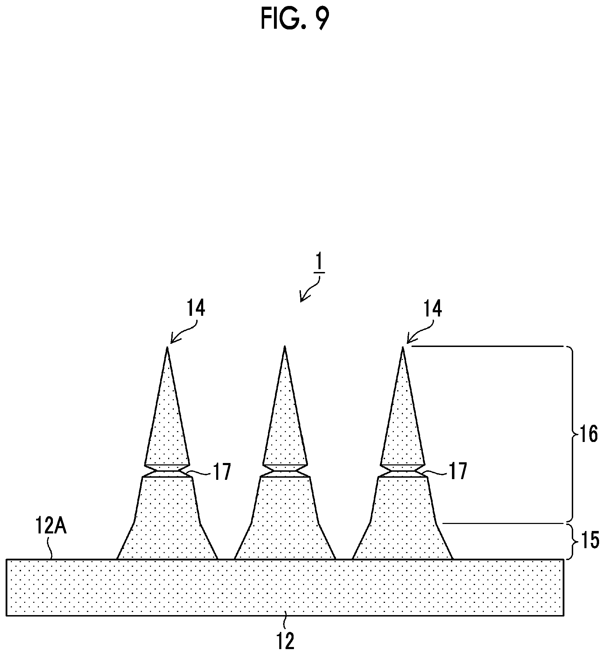

As shown in Fig. 9, a plate precursor 1 is manufactured from the base material 10 through the first cutting step (step S1) and the second cutting step (step S2). The plate precursor 1 comprises a base 12 and a plurality of needle-shaped protrusions 14 formed on a flat surface 12A of the base 12. The needle-shaped protrusion 14 comprises a frustum portion 15, a needle portion 16, and a constricted shape 17. The plurality of needle-shaped protrusions 14 are arranged at predetermined positions. As will be described below, the needle-shaped protrusions 14 are formed to coincide in size, shape, and arrangement with the needle-shaped protrusions 114 of the microneedle array 100 to be manufactured. The needle-shaped protrusion 14 may not comprise the frustum portion 15.

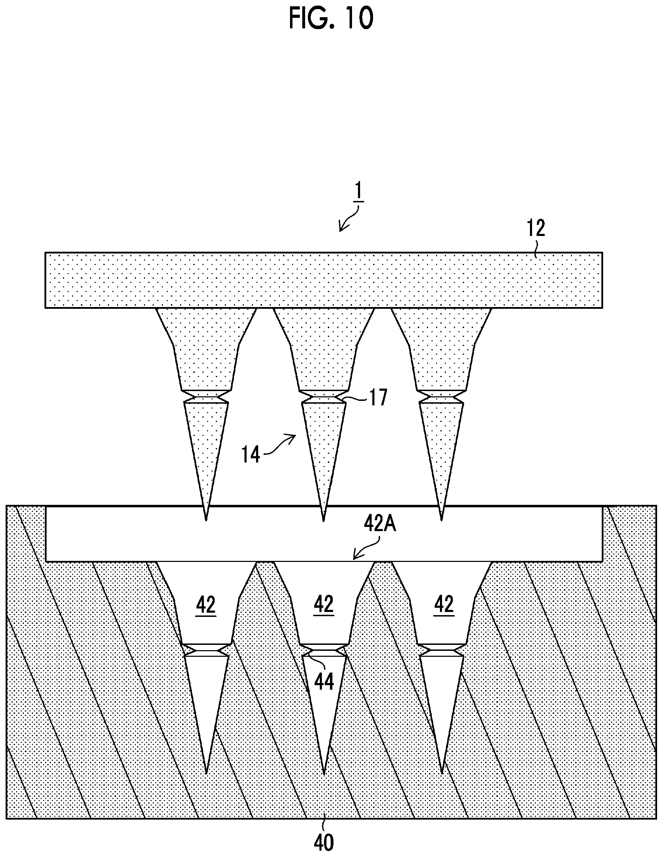

As shown in Fig. 10, a resin mold 40 having a plurality of needle-shaped holes 42 is manufactured from the plate precursor 1. The resin mold 40 is preferably made of silicone rubber. The resin mold 40 made of silicone rubber is manufactured by a liquid injection molding method. In the liquid injection molding method, a medical grade silicone material (for example, MDX4-4210 manufactured by Dow Corning) can be applied as a silicone rubber material for molding the resin mold 40 by setting the plate precursor 1 in an injection molding machine, injecting liquid silicone rubber, and curing the liquid silicone rubber.

After curing, the resin mold 40 is released from the plate precursor 1. Although the needle-shaped protrusion 14 has the constricted shape 17, the resin mold 40 deforms, so that the resin mold 40 can be released from the plate precursor 1.

The needle-shaped hole 42 is formed in an inverted shape of the needle-shaped protrusion 14. A protrusion shape 44 corresponding to the constricted shape 17 is formed in the needle-shaped hole 42. In the protrusion shape 44, the hole diameter of the needle-shaped hole 42 is narrowed from an opening 42A of the needle-shaped hole 42 to the tip.

### (First Array Manufacturing Step)

In the first array manufacturing step, the needle-shaped holes of the resin mold are filled with a drug solution and then dried (step S4).

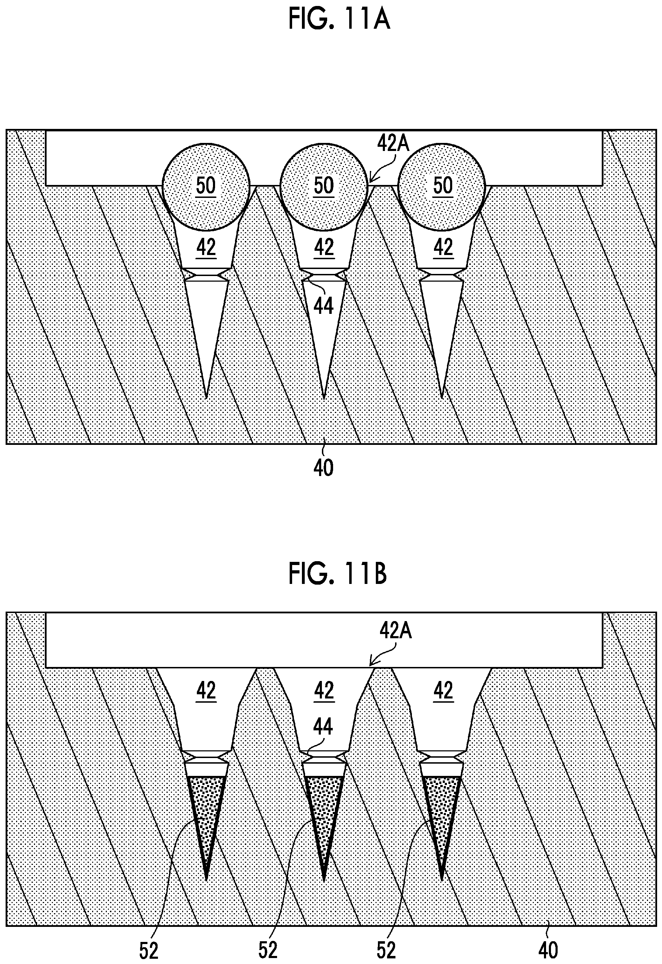

The first array manufacturing step is performed in a sterile room, for example, an isolator. As shown in Fig. 11A, a drug solution 50 containing the drug is discharged in a form of liquid droplet from a nozzle of a drug solution discharge head (not shown) for each needle-shaped hole 42 of the resin mold 40. The drug solution 50 closes the opening 42A of the needle-shaped hole 42.

As the drug solution discharge head, for example, an ink jet head such as a solenoid type ink jet head or a piezoelectric ink jet head can be used. The amount of liquid droplet of one drug solution 50 discharged from the nozzle is about 1 nL or more and 150 nL or less.

The drug is at least one selected from the group consisting of peptides, proteins, nucleic acids, polysaccharides, vaccines, pharmaceutical compounds, and cosmetic components. The drug solution 50 is preferably a water-soluble polymer, for example, polysaccharides (such as hyaluronic acid, sodium hyaluronate, pullulan, dextran, dextrin, sodium chondroitin sulfate, carboxymethyl cellulose, hydroxypropyl cellulose, hydroxyethyl starch, hydroxypropyl methyl cellulose, polyvinylpyrrolidone, polyoxyethylene polyoxypropylene glycol, polyethylene glycol, and arabic rubber) and proteins (such as gelatin). Note that the drug solution 50 is not limited thereto.

Next, a suction pump (not shown) is driven from an opposite surface of the opening 42A of the needle-shaped hole 42 of the resin mold 40 to move the drug solution 50 landed on the opening 42A to a tip portion from the protrusion shape 44 of the needle-shaped hole 42. By moving the drug solution 50 to the tip portion, the needle-shaped hole 42 is filled with the drug solution 50. In a case where the drug solution 50 can be moved to the tip portion, suction is not essential.

As shown in Fig. 11B, the drug solution 50 filling the tip portion of the needle-shaped hole 42 is dried to form a first layer 52 containing the drug. The drying is performed, for example, by blowing air on the drug solution 50 filling the needle-shaped hole 42. In addition, the drying is preferably performed in a drying chamber where the temperature and humidity can be adjusted. A drying speed of the drug solution 50 can be increased by reducing a pressure in the drying chamber.

As a method for filling the needle-shaped hole 42 with the drug solution 50, the needle-shaped hole 42 may be filled by supplying the drug solution 50 while bringing a tip portion of a slit nozzle into contact with the resin mold 40. In addition, the needle-shaped hole 42 may be filled with the drug solution 50 by supplying the drug solution 50 to the resin mold 40 by a dispenser or the like and bringing a blade into contact with the resin mold 40.

### (Second Array Manufacturing Step)

In the second array manufacturing step, after the first array manufacturing step (step S4), the needle-shaped holes are filled with a base material solution and dried (step S5).

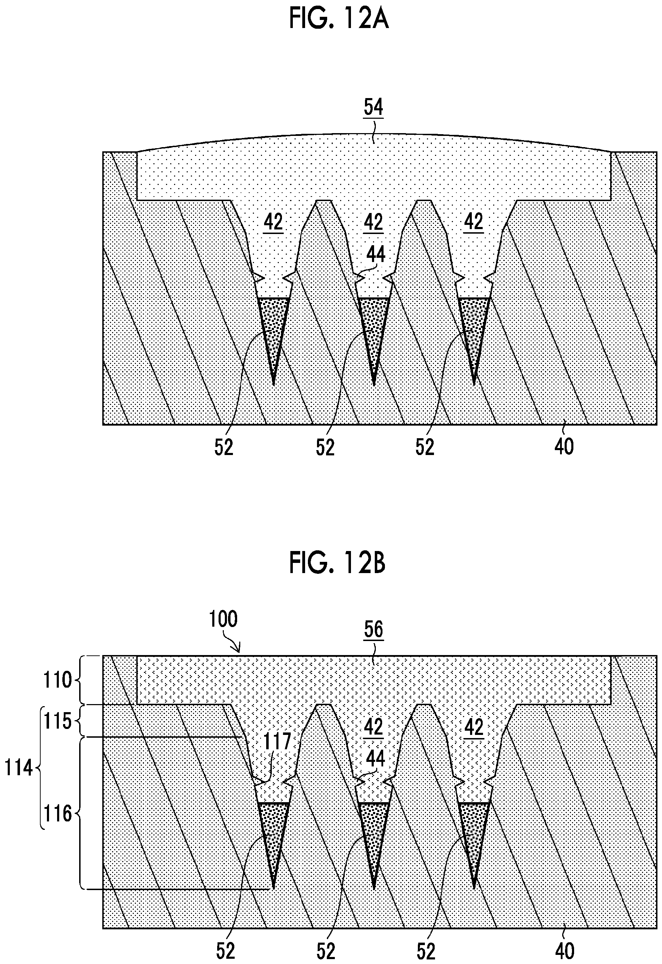

The second array manufacturing step is performed in a sterile room, for example, an isolator. As shown in Fig. 12A, a base material solution 54 containing no drug is spotted and applied from a nozzle of a spotting head (not shown). The spotting is performed by holding a certain amount of the base material solution 54 flowing out to the nozzle of the spotting head and bringing the held base material solution 54 into contact with a flat surface surrounded by a bank portion of the resin mold 40.

The base material solution 54 is preferably a water-soluble polymer, for example, polysaccharides (such as hyaluronic acid, sodium hyaluronate, pullulan, dextran, dextrin, sodium chondroitin sulfate, carboxymethyl cellulose, hydroxypropyl cellulose, hydroxyethyl starch, hydroxypropyl methyl cellulose, polyvinylpyrrolidone, polyoxyethylene polyoxypropylene glycol, polyethylene glycol, and arabic rubber) and proteins (such as gelatin). The drug solution 50 and the base material solution 54 may be the same as or different from each other. Note that the base material solution 54 is not limited thereto.

Next, in the same manner as in the first array manufacturing step, a suction pump (not shown) is driven to move the base material solution 54 spotted on a flat portion of the resin mold 40 to the needle-shaped hole 42. By moving the base material solution 54, the needle-shaped hole 42 is filled with the base material solution 54. In a case where the base material solution 54 can be moved, suction is not essential.

As shown in Fig. 12B, the base material solution 54 filling the needle-shaped hole 42 is dried to form a second layer 56 containing no drug. The drying is performed, for example, by blowing air on the base material solution 54 filling the needle-shaped hole 42. In addition, the drying is preferably performed in a drying chamber where the temperature and humidity can be adjusted. A drying speed of the base material solution 54 can be increased by reducing a pressure in the drying chamber.

The filling with the base material solution 54 is not limited to the spotting by the spotting head, and may be added dropwise by a dispenser.

The microneedle array 100 is manufactured by the resin mold 40 through the first array manufacturing step and the second array manufacturing step. The microneedle array 100 is composed of a plurality of needle-shaped protrusions 114 and a sheet 110. The needle-shaped protrusion 114 is composed of a frustum portion 115, a needle portion 116, and a constricted shape 117.

### (Peeling Step)

In the peeling step, the microneedle array manufactured by the resin mold is peeled from the resin mold (step S6).

As shown in Fig. 13, the microneedle array 100 is peeled from the resin mold 40. A peeling method is not particularly limited.

In the peeling method, for example, a base material having a pressure-sensitive adhesive layer can be adhered to a back surface of the microneedle array 100 (a surface on which the needle-shaped protrusion 114 is not formed), and the base material can be peeled to be turned over from an end portion. In addition, a method for installing a sucker on the above-mentioned base material and pulling up the microneedle array 100 perpendicularly to the resin mold 40 while sucking the base material with air can be applied. The peeling method is not particularly limited.

Although the protrusion shape 44 is formed in the needle-shaped hole 42 of the resin mold 40, the microneedle array 100 can be peeled from the resin mold 40 without being destroyed from the mechanical characteristics of the resin mold 40 and the microneedle array 100. The microneedle array 100 comprising the sheet 110 and the needle-shaped protrusion 114 composed of the frustum portion 115, the needle portion 116, and the constricted shape 117 can be manufactured. The constricted shape 117 is formed at an angle θ1 that is generally the same as the cutting edge angle θ of the second cutting tool 30.

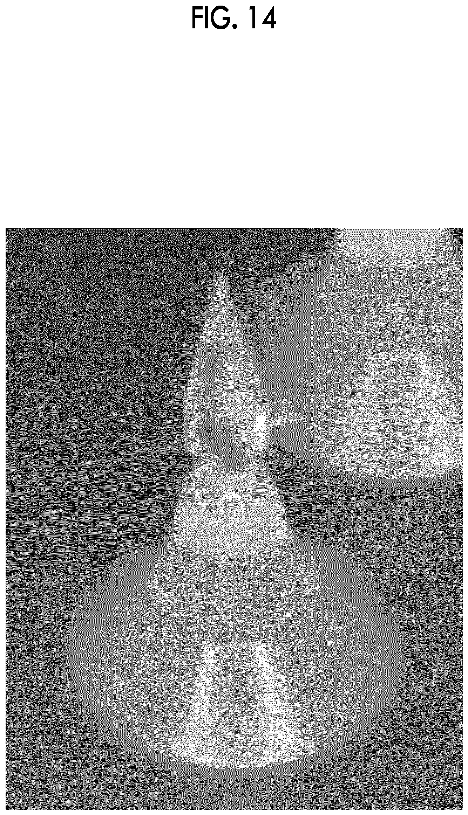

Fig. 14 is a photomicrograph of the needle-shaped protrusion of the microneedle array. According to the photomicrograph, it can be confirmed that the constricted shape is formed on the needle-shaped protrusion of the microneedle array.

### (Puncturing Property)

In order to confirm the puncturing property of the manufactured microneedle array, a plurality of types of microneedle arrays having different minimum diameters of the constricted shape were manufactured by the method for manufacturing a microneedle array of the embodiment of the present invention. Nine types of microneedle arrays having minimum diameters of the constricted shapes of (1) 80 µm, (2) 90 µm, (3) 100 µm, (4) 110 µm, (5) 120 µm, (6) 130 µm, (7) 140 µm, (8) 150 µm, and (9) 160 µm were manufactured. Nine types of microneedle arrays were manufactured only using the base material solution.

Nine types of microneedle arrays punctured the excised pig skin and hand-rubbed parallel to the skin for 10 seconds. In all the microneedle arrays, it was confirmed that the needle-shaped protrusions were broken in a constricted shape, and that the tip part from the constricted shape remained in the excised pig skin.

### Explanation of References

1: plate precursor
10: base material
10A: flat surface
12: base
12A: flat surface
14: needle-shaped protrusion
15: frustum portion
16: needle portion
17: constricted shape
20: first cutting tool
22: first blade
24: shank
30: second cutting tool
32: second blade
32A: outer edge
34: support member
40: resin mold
42: needle-shaped hole
42A: opening
44: protrusion shape
50: drug solution
52: first layer
54: base material solution
56: second layer
100: microneedle array
110: sheet
114: needle-shaped protrusion
115: frustum portion
116: needle portion
117: constricted shape
A1: tool axis
A2: tool axis
NA: axis

## Claims

1. A method for manufacturing a microneedle array, the method comprising:
a first cutting step of forming a needle-shaped protrusion by cutting a base material by a first cutting tool;
a second cutting step of forming a constricted shape in the needle-shaped protrusion by cutting a part of the needle-shaped protrusion by a second cutting tool different from the first cutting tool;
a mold forming step of molding, from a plate precursor manufactured from the base material through the first cutting step and the second cutting step, a resin mold having a needle-shaped hole which has an inverted shape of the plate precursor;
a first array manufacturing step of filling the needle-shaped hole of the resin mold with a drug solution and then drying the drug solution;
a second array manufacturing step of filling the needle-shaped hole with a base material solution and drying the base material solution, after the first array manufacturing step; and
a peeling step of peeling, from the resin mold, a microneedle array manufactured by the resin mold.

2. The method for manufacturing a microneedle array according to claim 1,
wherein a minimum diameter of the constricted shape is 80 µm or more and 140 µm.

3. The method for manufacturing a microneedle array according to claim 1 or 2,
wherein the resin mold is made of silicone rubber.

4. The method for manufacturing a microneedle array according to claim 3,
wherein the resin mold is molded by a liquid injection molding method.

5. The method for manufacturing a microneedle array according to any one of claims 1 to 4,
wherein the constricted shape is formed in a range of 300 µm or more and 500 µm or less from a tip of the needle-shaped protrusion.

6. The method for manufacturing a microneedle array according to any one of claims 1 to 5, wherein the second cutting tool has a cutting edge angle of 30° or more.

7. The method for manufacturing a microneedle array according to any one of claims 1 to 6, wherein the first cutting tool and the second cutting tool revolve while rotating.

8. The method for manufacturing a microneedle array according to any one of claims 1 to 7,
wherein a drug contained in the drug solution is at least one selected from the group consisting of a peptide, a protein, a nucleic acid, a polysaccharide, a vaccine, a pharmaceutical compound, and a cosmetic component.
